# FASCICULE DE BREVET EUROPEEN

(11) **EP 1 634 569 B1**
(45) Date de publication et mention de la délivrance du brevet: **24.12.2008**
(21) Numéro de dépôt: 05291659.0
(22) Date de dépôt: 03.08.2005
(51) Int. Cl.: A61K 8/34, A61K 8/41, A61Q 5/02, A61Q 5/12

(54) **Composition cosmétique à base d'un tensioactif cationique, d'un alcool gras, d'un polymère cationique non siliconé et d'un diol**
Kosmetische Zusammensetzung auf der Basis eines kationischen Tensids, eines Fettalkohols, eines kationischen nicht-silikonhaltigen Polymers und eines Diols
Cosmetic composition based on a cationic surfactant, a fatty alcohol, a cationic non-silicone polymer and a diol

(30) Priorité: 08.09.2004 FR 0451989
(43) Date de publication de la demande: 15.03.2006
(73) Titulaire: L'ORÉAL, 75008 Paris (FR)
(72) Inventeur: Lazzeri, Pascale, 92300 Levallois Perret (FR); Decoster, Sandrine, 95210 Saint Gratien (FR)
(74) Mandataire: Le Blainvaux Bellegarde, Françoise

(56) Documents cités:
- WO-A-99/62492
- US-A1- 2004 103 488

## Description

La présente invention est relative à de nouvelles compositions cosmétiques, notamment capillaires, comprenant au moins un tensioactif cationique particulier, au moins un polymère cationique, au moins un alcool gras, et au moins un diol particulier. Elle vise également l'utilisation de ces compositions en cosmétique, et en particulier l'utilisation de ces compositions comme après-shampooing.

Les après-shampooings classiques comprenant un tensioactif cationique et des alcools gras permettent d'obtenir de bonnes propriétés cosmétiques des cheveux. Toutefois, l'utilisateur a parfois la sensation que ses cheveux sont lourds, peu légers, et qu'ils regraissent facilement, tout particulièrement si ses cheveux sont fins, de plus pour certains types de cheveux les propriétés cosmétiques ne sont pas suffisantes. En outre, la répartition du produit est parfois difficile.

WO99/62492 décrit des compositions de conditionnement capillaire contenant des alcools gras, des tensioactifs cationiques, des polymères cationiques mais ne contenant pas de diol ayant 6 atomes de carbone.

La Demanderesse vient de découvrir de façon surprenante que des compositions comprenant au moins un tensioactif cationique particulier, au moins un polymère cationique non siliconé, au moins un ou plusieurs alcools gras avec une concentration totale en alcool(s) gras supérieure ou égale à 4% en poids par rapport au poids total de la composition, et au moins un diol ayant 6 atomes de carbone, permettaient de remédier aux inconvénients mentionnés ci-dessus.

Elle a en particulier constaté que ces compositions permettaient un assouplissement de la fibre, c'est-à-dire rendait la fibre moins rêche et plus malléable.

Elle a également constaté que lors de l'application, les compositions se répartissaient et s'étalaient facilement des racines jusqu'aux pointes, et que le rinçage était particulièrement aisé.

Elle a enfin remarqué que ces compositions conféraient de bonnes propriétés cosmétiques aux cheveux, et notamment le démêlage, le lissage, que les cheveux étaient légers et regraissaient moins vite, et que les cheveux se mettaient en forme très facilement, et durablement.

La présente invention a donc pour objet une composition cosmétique, notamment capillaire, caractérisée en ce qu'elle comprend, dans un milieu cosmétiquement acceptable, au moins un tensioactif cationique choisi parmi les sels d'ammonium quaternaire, au moins un polymère cationique non siliconé, au moins un alcools gras, et au moins un diol ayant 6 atomes de carbone, la concentration totale en alcool(s) gras étant supérieure ou égale à 4% en poids par rapport au poids total de la composition.

Un autre objet de l'invention concerne un procédé cosmétique capillaire mettant en oeuvre les compositions selon l'invention.

L'invention a également pour objet l'utilisation des compositions selon l'invention pour le traitement cosmétique des cheveux et du cuir chevelu.

Un autre objet de l'invention est l'utilisation de la composition pour le conditionnement des matières kératiniques telles que les cheveux.

D'autres caractéristiques, aspects, objets et avantages de l'invention apparaîtront encore plus clairement à la lecture de la description et des exemples qui suivent.

Par « au moins un », on comprendra « un ou plusieurs », c'est-à-dire un, deux, trois ou plus.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

Le milieu cosmétiquement acceptable est de préférence constitué par de l'eau ou par un ou plusieurs solvants cosmétiquement acceptable de préférence hydrophile ou par des mélanges eau-solvant(s). Ces solvants sont de préférence des alcools et notamment les monoalcools, linéaires ou ramifiés en C1-C6, comme l'éthanol, le tertiobutanol, le n-butanol, l'isopropanol ou le n-propanol.

L'eau ou le mélange d'eau et de solvants cosmétiquement acceptable peut être présent dans la composition selon l'invention en une teneur allant de 30% à 99% en poids, par rapport au poids total de la composition, et de préférence de 60% à 90% en poids.

Parmi les sels d'ammonium quaternaire on peut citer les sels d'alkylpyridinium, les sels d'ammonium d'imidazoline, les sels de diammonium quaternaire, les sels d'ammonium contenant au moins une fonction ester.

Les tensioactifs cationiques sont de préférence monomériques, c'est-à-dire que ni le cation, ni l'anion ne sont des oligomères ou des polymères.

A titre de sels d'ammonium quaternaire, on peut notamment citer, par exemple :
- ceux qui présentent la formule générale (V) suivante : 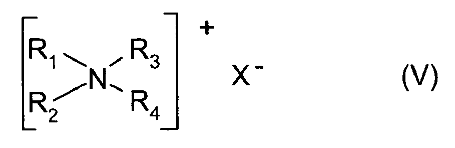 dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, notamment en C12-C22, alcoxy, polyoxyalkylène (C2-C6), alkylamide, alkyl(C12-C22)amidoalkyle(C2-C6), alkyl(C12-C22)acétate, hydroxyalkyle, comportant environ de 1 à 30 atomes de carbone ; X- est un anion choisi dans le groupe des halogénures, phosphates, acétates, lactates, alkyl(C2-C6)sulfates, alkyl- ou alkylaryl-sulfonates ;
- les sels d'ammonium quaternaire de l'imidazoline comme, par exemple, ceux de formule (VI) suivante : 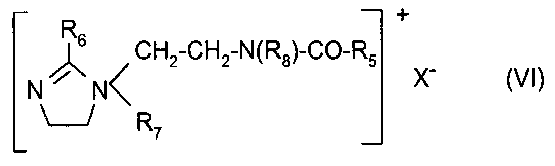 dans laquelle R5 représente un radical alcényle ou alkyle comportant de 8 à 30 atomes de carbone par exemple dérivés des acides gras du suif ou du coprah, R6 représente un atome d'hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates. De préférence, R5 et R6 désignent un mélange de radicaux alcényle ou alkyle comportant de 12 à 21 atomes de carbone par exemple dérivés des acides gras du suif, R7 désigne méthyle, R8 désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (VII) : 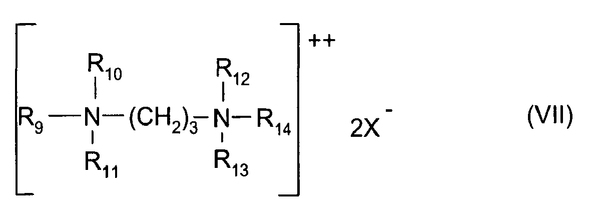 dans laquelle R9 désigne un radical aliphatique comportant environ de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (VIII) suivante : 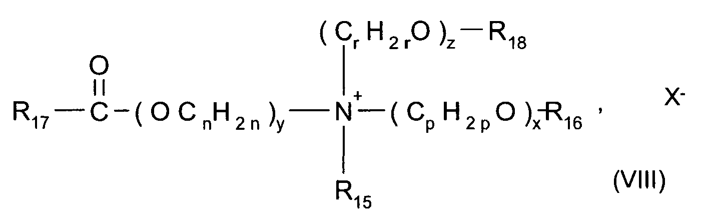 dans laquelle :
   R15 est choisi parmi les radicaux alkyles en C1-C6 et les radicaux hydroxyalkyles ou dihydroxyalkyles en C1-C6 ;
   R16 est choisi parmi :
   - le radical 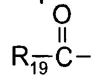
   - les radicaux R20 hydrocarbonés en C1-C22, linéaires ou ramifiés, saturés ou insaturés,
   - l'atome d'hydrogène,
   R17 est choisi parmi :
   - le radical 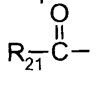
   - les radicaux R22 hydrocarbonés en C1-C6, linéaires ou ramifiés, saturés ou insaturés,
   - l'atome d'hydrogène,
   R17, R19 et R21, identiques ou différents, sont choisis parmi les radicaux hydrocarbonés en C7-C21, linéaires ou ramifiés, saturés ou insaturés ;
   r, n et p, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

Les radicaux alkyles R15 peuvent être linéaires ou ramifiés, et plus particulièrement linéaires.

De préférence, R15 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R16 est un radical R20 hydrocarboné, il peut être long et avoir de 12 à 22 atomes de carbone, ou court et avoir de 1 à 3 atomes de carbone.

Lorsque R18 est un radical R22 hydrocarboné, il a de préférence 1 à 3 atomes de carbone.

Avantageusement, R17, R19 et R21, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (VIII) dans laquelle :
- R15 désigne un radical méthyle ou éthyle,
- x et y sont égaux à 1 ;
- z est égal à 0 ou 1 ;
- r, n et pt sont égaux à 2 ;
R16 est choisi parmi :
- le radical 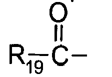
- les radicaux méthyle, éthyle ou hydrocarbonés en C14-C22,
- l'atome d'hydrogène ;
- R18 est choisi parmi :

- le radical 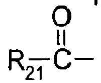
- l'atome d'hydrogène ;
- R17, R19 et R21, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les sels (et notamment les méthosulfates) de dipalmitoyléthylhydroxyéthylméthylammonium, les sels (et notamment les chlorures) de tétraalkylammonium comme, par exemple, les sels (et notamment les chlorures) de dialkyldiméthylammonium ou d'alkyltriméthyl-ammonium dans lesquels le radical alkyle comporte environ de 12 à 22 atomes de carbone, en particulier les sels (et notamment les chlorures) de béhényltriméthylammonium, de distéaryldiméthylammonium, de cétyltriméthylammonium, de benzyldiméthylstéarylammonium ou encore, d'autre part, les sels (et notamment les chlorures) de palmitylamidopropyltriméthylammonium ou les sels (et notamment les chlorures) de stéaramidopropyldiméthyl-(myristylacétate)-ammonium, et notamment le chlorure de stéaramidopropyldiméthyl-(myristyl acétate)-ammonium commercialisé sous la dénomination CERAPHYL® 70 par la société VAN DYK.

Les tensioactifs cationiques encore plus particulièrement préférés dans la composition de l'invention sont choisis parmi les sels d'ammonium quaternaire, et en particulier parmi le chlorure de béhényltriméthylammonium, le chlorure de cétyl triméthyl ammonium, le méthosulfate de dipalmitoyléthyl hydroxyéthyl méthyl ammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

Le ou les tensioactifs cationiques peuvent être présents dans la composition à une teneur allant de 0,01 à 10% en poids, de préférence de 0,1 à 5% en poids, encore de préférence de 0,2 à 4% en poids du poids total de la composition.

L'alcool gras selon l'invention peut linéaire ou ramifié, saturé ou insaturé, et comporter de 8 à 40 atomes de carbone.

L'alcool gras peut être oxyalkyléné ou glycérolé.

L'alcool gras peut présenter de préférence la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en C8-C40 de préférence en C12-C24 ou alkényle en C8-C40, de préférence en C12-C24. R peut être substitué par un ou plusieurs groupements hydroxy et notamment par un ou deux groupements hydroxy.

A titre d'exemple on peut citer l'alcool cétylique, l'alcool stéarylique, l'alcool béhénylique, l'alcool isocétylique, l'alcool isostéarylique, l'alcool isobéhénylique et l'alcool oléique et leurs mélanges. De préférence, l'alcool est choisi parmi l'alcool cétylique, l'alcool stéarylique, l'alcool béhénylique et leurs mélanges.

Par alcool gras oxyalkyléné selon l'invention, on entend tout alcool gras pur de structure suivante : 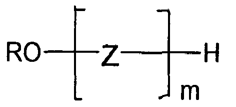 dans laquelle :
R désigne un radical saturé ou insaturé, linéaire ou ramifié, comportant de 8 à 40 atomes de carbone et de préférence de 8 à 30,
Z représente un radical oxyéthyléné (i) et/ou oxypropyléné (ii)1 et (ii)2 de formules respectives suivantes :

   ― CH₂―CH₂―O― (i) 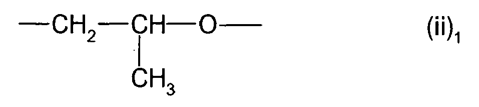 ― CH₂―CH₂―CH₂―O― (ii)₂
m représente le nombre de groupements oxyde d'éthylène (i) et/ou oxyde de propylène (ii)1 ou (ii)2, allant de 1 à 250 et de préférence de 2 à 100.

Par alcool gras glycérolé, on entend tout alcool gras pur de structure suivante : 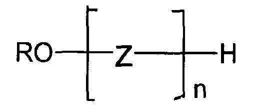 dans laquelle,
R désigne un radical saturé ou insaturé, linéaire ou ramifié, comportant de 8 à 40 atomes de carbone et de préférence de 8 à 30,
Z représente un radical glycérolé (iii) de formule suivante : 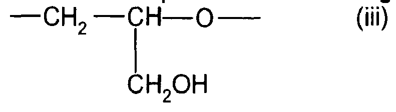 n représente le nombre de groupements glycérol (iii) et est compris entre 1 et 30 et de préférence entre 1 et 10.

Des alcools gras oxyalkylénés particulièrement préférés selon l'invention sont des alcools gras, saturés ou non, linéaires ou ramifiés, comportant de 10 à 20 atomes de carbone et 2 à 40 groupements oxyde d'éthylène.

Comme composés de type alcool gras oxyalkyléné, on peut notamment citer les produits commercialisés suivants :
Mergital LM2 (COGNIS) [alcool laurique 2 OE] ;
Ifralan L12 (IFRACHEM) et Rewopal 12 (GOLDSCHMIDT) [alcool laurique 12 OE] ;
Empilan KA 2.5/90FL (ALBRIGHT & WILSON) et Mergital BL309 (COGNIS) [alcool décylique 3 OE] ;
Empilan KA 5/90 FL (ALBRIGHT & WILSON) et Mergital BL589 (COGNIS) [alcool décylique 5 OE] ;
Brij 58 (UNIQUEMA) et Simulsol 58 (SEPRIC) [alcool cétylique 20 OE] ;
Emulgin 05 (COGNIS) [alcool oléocétylique 5 OE] ;
Mergital OC30 (COGNIS) [alcool oléocétylique 30 OE] ;
Brij 72 (UNIQUEMA) [alcool stéarylique 2 OE] ;
Brij 76 (UNIQUEMA) [alcool stéarylique 10 OE] ;
Brij 78P (UNIQUEMA) [alcool stéarylique 20 OE] ;
Brij 700 (UNIQUEMA) [alcool stéarylique 100 OE] ;
Emulgin B1 (COGNIS) [alcool cétylstéarylique 12 OE] ;
Emulgin L (COGNIS) [alcool cétylique 9 OE et 2 OP] ;
Witconol APM (GOLDSCHMIT) [alcool myristique 3 OP] ;
Comme composés de type alcool gras glycérolé, on peut notamment citer l'alcool laurique à 4 moles de glycérol (nom INPCI : POLYGLYCERYL-4 LAURYL ETHER), l'alcool oléique à 4 moles de glycérol (nom INPCI: POLYGLYCERYL-4 OLEYL ETHER), l'alcool oléique à 2 moles de glycérol (nom INPCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool gras peut représenter un mélange d'alcools gras, ce qui signifie que dans un produit commercial peuvent coexister plusieurs espèces d'alcools gras, sous forme d'un mélange.

Avantageusement, l'alcool gras est solide ou pâteux à la température de 20°C. Par « alcool gras solide ou pâteux à 20°C », on entend au sens de la présente invention un alcool gras présentant une viscosité mesurée avec un rhéomètre avec un taux de cisaillement de 1s⁻¹ supérieure ou égale à 1 Pa.s.

De préférence, les alcools gras de l'invention sont non oxyalkylénés et/ou non glycérolés.

Le ou les alcools gras peuvent être présents dans la composition à une teneur allant de 4 à 15%, de préférence de 4,5 à 10%, encore de préférence de 4,5 à 8% en poids du poids total de la composition.

Par diol ayant 6 atomes de carbone au sens de l'invention, on entend tout composé hydrocarboné comprenant 2 fonctions hydroxy et 6 atomes de carbone.

Le diol peut être linéaire ou ramifié et comprendre des fonctions éthers.

Le diol est de préférence choisi parmi l'hexane diol-1,6, le dipropylèneglycol et leurs mélanges.

Le ou les diols peuvent être présents dans la composition à une teneur allant de 0,1 à 10%, de préférence de 0,25 à 5%, encore de préférence de 0,5 à 3% en poids par rapport au poids total de la composition.

Selon l'invention, les compositions comprennent en outre au moins un polymère cationique non siliconé. Par non siliconé, on entend selon la présente invention que le polymère cationique ne comprend pas de motifs (Si-O).

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 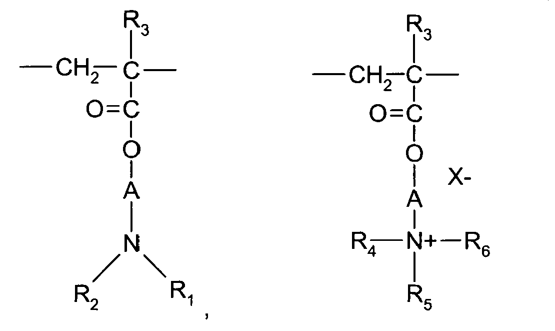 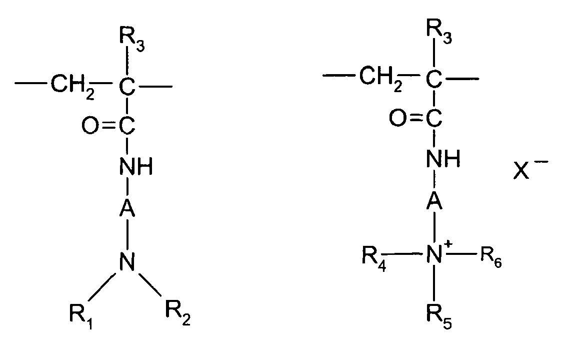 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de monomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymères d'acrylamide et de méthosulfate de méthacryloyloxy-éthyltriméthylammonium vendus sous la dénomination RETEN par la société HERCULES,
   - les copolymères vinylpyrrolidone / acrylate ou méthacrylate de dialkylaminoalkyle quaternisés ou non. Ces polymères sont décrits en détail dans les brevets français 2.077.143 et 2.393.573,
   - les terpolymères méthacrylate de diméthyl amino éthyle/vinylcaprolactame/ vinylpyrrolidone,
   - les copolymères vinylpyrrolidone / méthacrylamidopropyl dimethylamine.
   - et les copolymères vinylpyrrolidone / méthacrylamide de diméthylamino-propyle quaternisé.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "HERCOSETT 57" par la société Hercules Inc. par la société HERCULES dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (1) ou (l') : 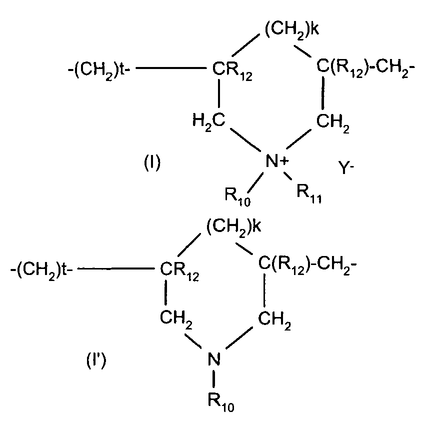 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide
(8) les polymères de diammonium quaternaire contenant des motifs récurrents répondant à la formule : 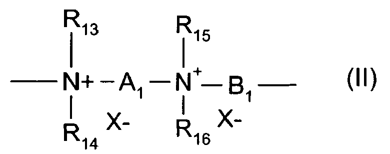 formule (II) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ; n désu=igne un entier allant de 1 à 8, de préférence de 2 à 4
   De préférence, X- est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 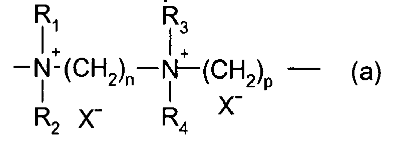 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature lNCl (CTFA).
(9) les polymères de polyammonium quaternaires comprenant des motifs de formule (III): 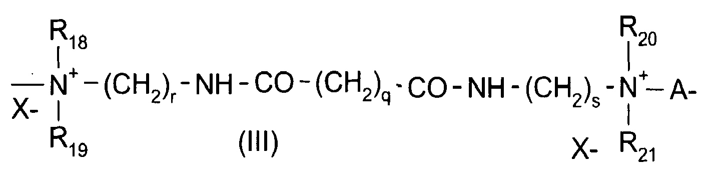 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical d'un dihalogénure ou représente de préférence -CH₂-CH₂-O-CH₂-CH₂-
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polymères éventuellement réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁₋C₄)ammonium, le pyrrolidone-carboxylate de chitosane et leurs mélanges.

Selon l'invention, le ou les polymères cationiques peuvent représenter de 0,001 % à 20 % en poids, de préférence de 0,01 % à 10% en poids et plus particulièrement de 0,02 à 5% en poids par rapport au poids total de la composition finale.

Le rapport des concentrations en poids tensioactif cationique /diol est de préférence supérieur à 1 et va de préférence de 1,5 à 10 et plus particulièrement de 2 à 5.

De préférence, le rapport des concentrations en poids alcool gras/tensioactif cationique est supérieur ou égal à 1, va de préférence de 1 à 10 et plus particulièrement de 1 à 5.

Le rapport des concentrations en poids tensioactif cationique/silicone va de préférence de 0,85 à 10 et plus particulièrement de 0,85 à 5.

Le rapport des concentrations en poids alcool gras/silicone va de préférence de 2 à 15 et plus particulièrement de 2 à 5.

La composition selon l'invention peut contenir en outre au moins un ou plusieurs additifs choisis parmi les parfums, les filtres UV, les tensioactifs non ioniques, anioniques, amphotères ou zwitterioniques, les conservateurs, les protéines, les vitamines, les provitamines, les polymères non ioniques, anioniques, cationiques, amphotères ou zwitterioniques, les huiles minérales, végétales ou synthétiques, les silicones, les cires végétales, les céramides, et tout autre additif classiquement utilisé dans les compositions cosmétiques, tels que les agents antipelliculaires, les agents anti-chute des cheveux, les colorants, les pigments, les réducteurs, les polyols autres que ceux de l'invention.

Ces additifs sont présents dans la composition à des teneurs avantageusement comprises entre 0,001 et 20% en poids du poids total de la composition. La quantité précise de chaque additif est fonction de sa nature et est déterminée facilement par l'homme du métier, et dépendra de l'application capillaire choisie.

De préférence, les compositions selon l'invention comprennent au moins une silicone et plus particulièrement une silicone aminée. La silicone peut être choisie parmi les amodiméthicones et les triméthylsilylamodiméthicones, les silicones à groupements ammonium quaternaire.

La silicone est de préférence présente dans la composition à une teneur allant de 0,01 à 6%, de préférence de 0,1 à 3%, et encore de préférence de 0,4 à 2% en poids du poids total de la composition.

L'homme du métier veillera à choisir les éventuels additifs et leur quantité de manière à ce qu'ils ne nuisent pas aux propriétés des compositions de la présente invention.

Les compositions selon l'invention peuvent se présenter sous forme de liquides fluides ou épaissis, de gels, de crèmes, ou d'émulsions simples ou multiples. Les compositions de traitement cosmétique des matières kératiniques conformes à l'invention peuvent aussi se présenter sous forme d'une mousse et peuvent être utilisées en application rincée ou non. Dans ce cas, elles peuvent être conditionnées dans un dispositif aérosol.

Les compositions peuvent être utilisées, par exemple, dans des shampoings, des après-shampooings, produits de coloration ou de décoloration ou de permanente, produits de coiffage, soins rincés, masques de soin profond, gels douches, lotions ou crèmes de traitement du cuir chevelu, ou encore déposées sur des lingettes.

De préférence, la composition selon l'invention est une composition non colorante. Par « composition non colorante », on entend une composition ne contenant essentiellement pas de colorant d'oxydation, c'est-à-dire moins de 0,5% en poids par rapport au poids total de la composition, de préférence moins de 0,1% en poids et plus particulièrement ne contenant pas de colorant d'oxydation.

L'invention a également pour objet l'utilisation des compositions selon l'invention pour le traitement cosmétique des cheveux et du cuir chevelu.

Un autre objet de l'invention est l'utilisation de la composition pour le conditionnement des matières kératiniques telles que les cheveux.

La présente invention a également pour objet un procédé de traitement cosmétique dans lequel la composition cosmétique selon l'invention est appliquée sur les cheveux, humides ou secs, cette application étant éventuellement suivie d'un rinçage. L'application de la composition s'effectue avantageusement après un shampooing.

Dans une forme de réalisation préférée de l'invention, les compositions selon l'invention sont utilisées comme après-shampooings pour le traitement des cheveux et du cuir chevelu.

Elles sont appliquées, dans ce cas-là, sur des cheveux humides ou secs, dans des quantités efficaces pour les traiter, cette application étant suivie d'un rinçage.

Les exemples suivants sont destinés à illustrer l'invention. Les quantités sont exprimées en matière active sauf indication contraire.

### Exemple 1

On a préparé une composition A d'après shampooing selon invention et une composition B non-conforme à l'invention:

| | Composition A | Composition B |
|---|---|---|
| Alcool cétylstéarylique | 7 g | 7 g |
| Chlorure de béhényl triméthyl ammonium | 3g MA | 3 gMA |
| Hexanediol-1,6 | 1 g | - |
| Isopropanol | - | 1 g |
| Mélange de Myristate/Palmitate/Stéarate de Myristyle/Cétyle/Stéaryle | 2,5 g | 2,5 g |
| Myristate d'isopropyle | 1,05 g | 1,05 g |
| Polydiméthylsiloxane à groupement aminopropyle en mélange avec un polydiméthylsiloxane (KF8020 de SHIN ETSU) | 0,1 gMA | 0,1 gMA |
| Homopolymère de chlorure méthacrylate d'éthyl triméthyl ammonium (SALCARE SC 95 de CIBA) | 0,15 gMA | 0,15 gMA |
| Parfum, Conservateurs | qs | qs |
| Agent de pH qs | pH 4 ± 0,5 | pH 4 ± 0,5 |
| Eau | Qsp 100g | Qsp 100g |

Les compositions A et B sont appliquées sur les cheveux, cette application étant suivie d'un rinçage.

Le rinçage de la composition A se fait plus facilement.

On observe que les cheveux traités avec la composition A selon l'invention sont plus souples, plus nerveux et plus lisses que les cheveux traités avec la composition B.

### Exemple 2

On a préparé une composition A d'après shampooing selon invention et une composition B non-conforme à l'invention:

| | Composition A | Composition B |
|---|---|---|
| Alcool cétylstéarylique | 5 g | 5 g |
| Chlorure de béhényl triméthyl ammonium | 3 gMA | 3 gMA |
| Hexanediol-1,6 | 1 g | - |
| Isopropanol | - | 1 g |
| Mélange de Myristate /Palmitate/Stéarate de Myristyle/Cétyle/Stéaryle | 1 g | 1g |
| Myristate d'isopropyle | 1,05 g | 1,05 g |
| Polydiméthylsiloxane à groupement aminopropyle en mélange avec un polydiméthylsiloxane (KF8020 de SHIN ETSU) | 0,5 gMA | 0,5 gMA |
| Homopolymère de chlorure méthacrylate d'éthyl triméthyl ammonium (SALCARE SC 95 de CIBA) | 0,3 gMA | 0,3 gMA |
| Parfum, Conservateurs | qs | qs |
| Agent de pH qs | pH 4 ± 0,5 | pH 4 ± 0,5 |
| Eau | Qsp 100g | Qsp 100g |

Les compositions A et B sont appliquées sur les cheveux, cette application étant suivie d'un rinçage.

Le rinçage de la composition A se fait plus facilement.

On observe que les cheveux traités avec la composition A selon l'invention sont plus souples, plus nerveux et plus lisses que les cheveux traités avec la composition B.

### Exemple 3

On a préparé une composition A d'après shampooing selon invention :

| | Composition A |
|---|---|
| Alcool cétylstéarylique | 7 g |
| Chlorure de béhényl triméthyl ammonium | 3gMA |
| Hexanediol-1,6 | 1 g |
| Dipropylèneglycol | 1 g |
| Mélange de Myristate/Palmitate/Stéarate de Myristyle/Cétyle/Stéaryle | 2,5 g |
| Myristate d'isopropyle | 1,05 g |
| Polydiméthylsiloxane à groupement aminopropyle en mélange avec un polydiméthylsiloxane (KF8020 de SHIN ETSU) | 0,5 gMA |
| Homopolymère de chlorure méthacrylate d'éthyl triméthyl ammonium (SALCARE SC 95 de CIBA) | 0,3 gMA |
| Parfum, Conservateurs | qs |
| Agent de pH qs | pH 4 ± 0,5 |
| Eau | Qsp 100g |

La composition s'étale facilement sur les cheveux.

On observe que les cheveux traités avec la composition A selon l'invention sont souples, nerveux et lisses

### Exemple 4

On a préparé une composition A d'après shampooing selon invention et une composition B non-conforme à l'invention:

| | Composition A | Composition B |
|---|---|---|
| Alcool cétylique | 4,5 g | 4,5 g |
| Chlorure de cétyl triméthyl ammonium | 3 g MA | 3 g MA |
| Hexanediol-1,6 | 1 gMA | - |
| lsopropanol | - | 1 gMA |
| Mélange de myristate /palmitate/stéarate de myristyle/cétyle/stéaryle | 2,5 g | 2,5 g |
| Polydiméthylsiloxane à groupement aminoéthyliminobutyle en émulsion aqueuse à 58% (DC2-8299 de DOW CORNING) | 0,5 g MA | 0,5 g MA |
| Stéarate de glycéryle | 1 g | 1 g |
| Parfum, Conservateurs | qs | qs |
| Agent de pH qs | pH 4 ± 0,5 | pH 4 ± 0,5 |
| Eau | Qsp 100g | Qsp 100g |

## Revendications

1. Composition cosmétique, notamment capillaire, **caractérisée en ce qu'**elle comprend, dans un milieu cosmétiquement acceptable, au moins un tensioactif cationique choisi parmi les sels d'ammonium quaternaire, au moins un alcools gras, éventuellement oxyéthyléné ou glycérolé au moins un polymère cationique non siliconé et au moins un diol ayant 6 atomes de carbone, la concentration totale en alcool(s) gras étant supérieure ou égale à 4% en poids par rapport au poids total de la composition le rapport des concentrations en poids tensioactif cationique/diol étant supérieur ou égal à 1.

2. Composition selon la revendication 1, **caractérisée en ce que** les sels d'ammonium quaternaire sont choisis parmi :
- ceux qui présentent la formule générale (V) suivante : 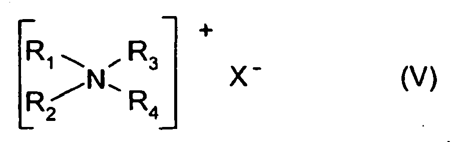
dans laquelle les symboles R1 à R4, qui peuvent être identiques ou différents, représentent un radical aliphatique, linéaire ou ramifié, comportant de 1 à 30 atomes de carbone, ou un radical aromatique tel que aryle ou alkylaryle ; X- est un anion choisi dans le groupe des halogénures, phosphates, acétates, lactates, alkyl(C2-C6)sulfates, alkyl- ou alkylarylsulfonates ;
- les sels d'ammonium quaternaire de l'imidazoline de formule (VI) suivante : 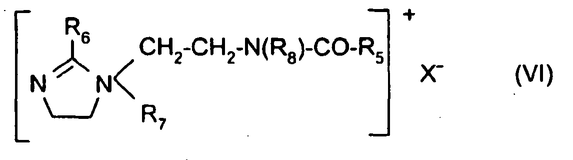
dans laquelle R5 représente un radical alcényle ou alkyle comportant de 8 à 30 atomes de carbone, R6 représente un atome d'hydrogène, un radical alkyle en C1-C4 ou un radical alcényle ou alkyle comportant de 8 à 30 atomes de carbone, R7 représente un radical alkyle en C1-C4 , R8 représente un atome d'hydrogène, un radical alkyle en C1-C4, X est un anion choisi dans le groupe des halogénures, phosphates, acétates, lactates, alkylsulfates, alkyl-ou-alkylarylsulfonates,
- les sels de diammonium quaternaire de formule (VII) : 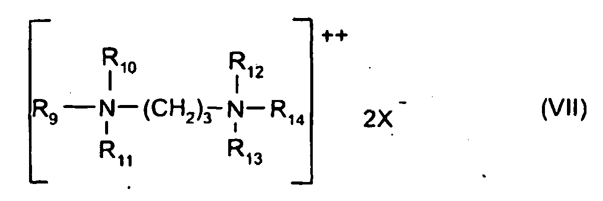
dans laquelle R9 désigne un radical aliphatique comportant de 16 à 30 atomes de carbone, R10, R11, R12, R13 et R14, identiques ou différents sont choisis parmi l'hydrogène ou un radical alkyle comportant de 1 à 4 atomes de carbone, et X- est un anion choisi dans le groupe des halogénures, acétates, phosphates, nitrates, éthylsulfates et méthylsulfates ;
- les sels d'ammonium quaternaire contenant au moins une fonction ester, de formule (VIII) suivante : 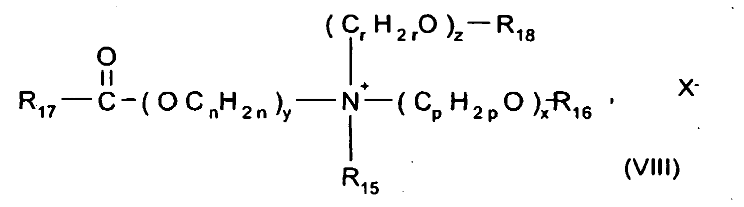
dans laquelle :
R15 est choisi parmi les radicaux alkyles en C1-C6 et les radicaux hydroxyalkyles ou dihydroxyalkyles en C1-C6 ;
R16 est choisi parmi :
- le radical 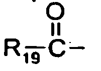
- les radicaux R20 hydrocarbonés en C1-C22, linéaires ou ramifiés, saturés ou insaturés,
- l'atome d'hydrogène,
R17 est choisi parmi :
- le radical 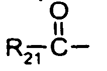
- les radicaux R22 hydrocarbonés en C1-C6, linéaires ou ramifiés, saturés ou insaturés,
- l'atome d'hydrogène, R17, R19 et R21, identiques ou différents, sont choisis parmi les radicaux hydrocarbonés en C7-C21, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X- est un anion simple ou complexe, organique ou inorganique ;
sous réserve que la somme x + y + z vaut de 1 à 15, que lorsque x vaut 0 alors R16 désigne R20 et que lorsque z vaut 0 alors R18 désigne R22.

3. Composition selon la revendication 2, **caractérisée en ce que** le composé de formule (VIII) est choisi parmi les sels de diacyloxyéthyl-diméthylammonium, de diacyloxyéthyl-hydroxyéthyl-méthylammonium, de monoacyloxyéthyl-dihydroxyéthyl-méthylammonium, de triacyloxyéthylméthylammonium, de monoacyloxyéthyl-hydroxyéthyl-diméthylammonium et leurs mélanges.

4. Composition selon la revendication 2, **caractérisée en ce que** le tensioactif de formule (V) est choisi parmi les sels de dipalmitoyléthylhydroxyéthylméthylammonium, les sels de béhényltriméthylammonium, de distéaryldiméthylammonium, de cétyl triméthylammonium, de benzyldiméthylstéarylammonium, les sels de palmitylamidopropyltriméthylammonium, les sels de stéaramido propyl diméthyl-(myristylacétate)-ammonium.

5. Composition selon la revendication 1, **caractérisée en ce que** le tensioactif cationique est choisi parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

6. Composition selon l'une quelconque des revendications précédentes, **caractérisée en ce que** le tensioactif cationique est présent dans la composition à une teneur allant de 0,01 à 10%, de préférence de 0,1 à 5%, et encore de préférence de 0,2 à 4% en poids du poids total de la composition.

7. Composition selon l'une quelconque des revendications précédentes, **caractérisée en ce que** l'alcool gras est linéaire ou ramifié, saturé ou insaturé.

8. Composition selon la revendication 7, **caractérisé en ce que** l'alcool gras présente la structure R-OH, dans laquelle R désigne un radical saturé ou insaturé, linéaire ou ramifié, comportant de 8 à 40 atomes de carbone et de préférence de 8 à 30.

9. Composition selon la revendication 8, **caractérisé en ce que** l'alcool gras est choisi parmi l'alcool cétylique, l'alcool stéarylique, l'alcool oléique et leurs mélanges.

10. Composition selon l'une quelconque des revendications précédentes, **caractérisée en ce que** l'alcool gras est solide ou pâteux à température ambiante.

11. Composition selon l'une quelconque des revendications précédentes, **caractérisée en ce que** l'alcool gras est présent dans la composition à une teneur allant de 4 à 15%, de préférence de 4,5 à 10%, encore de préférence de 4,5 à 8 % en poids du poids total de la composition.

12. Composition selon l'une quelconque des revendications précédentes, **caractérisée en ce que** le diol est choisi parmi l'hexanediol-1,6, le dipropylèneglycol et leurs mélanges.

13. Composition selon l'une quelconque des revendications précédentes, **caractérisée en ce que** le diol est présent dans la composition à une teneur allant de 0,1 à 10%, de préférence de 0,25 à 5% et encore de préférence de 0,5 à 3% en poids du poids total de la composition.

14. Composition selon l'une quelconque des revendications 1 à 13, **caractérisée par le fait que** le polymère cationique est choisi parmi :
(1) les homopolymères ou copolymères dérivés d'esters ou d'amides acryliques ou méthacryliques et comportant au moins un des motifs de formules suivantes: 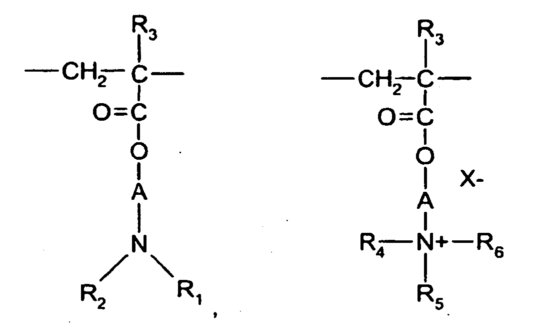 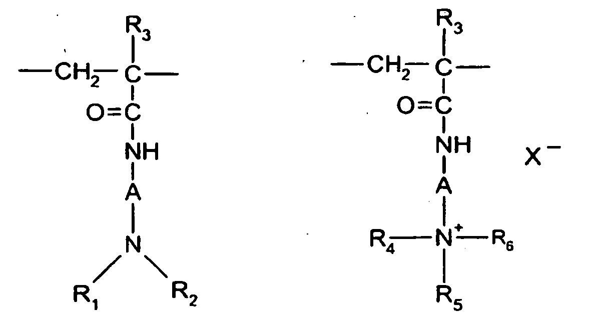 dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
R₁ et R₂, identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.
(2) Les polysaccharides cationiques
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères
(4) les polyaminoamides solubles dans l'eau préparés en particulier par polycondensation d'un composé acide avec une polyamine ;
(5) les dérivés de polyaminoamides résultant de la condensation de polyalcoylènes polyamines avec des acides polycarboxyliques suivie d'une alcoylation par des agents bifonctionnels
(6) les polymères obtenus par réaction d'une polyalkylène polyamine comportant deux groupements amine primaire et au moins un groupement amine secondaire avec un acide dicarboxylique
(7) les cyclopolymères d'alkyl diallyl amine ou de dialkyl diallyl ammonium
(8) les polymères de diammonium quaternaire contenant des motifs récurrents répondant à la formule : 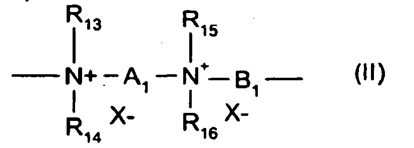 formule (II) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou - CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;
X⁻ est un anion
(9) les polymères de polyammonium quaternaires comprenant des motifs de formule (III): 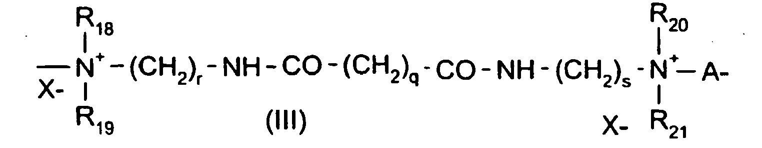 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X⁻ désigne un anion tel qu'un halogènure,
A désigne un radical d'un dihalogénure ou représente de préférence -CH₂-CH₂-O-CH₂-CH₂-.
(10) Les polymères quaternaires de vinylpyrrolidone et de vinylimidazole ,
(11) Les polymères réticulés de sels de méthacryloyloxyalkyl(C1-C4) trialkyl(C1-C4)ammonium,
(12) lesprotéines cationiques ou des hydrolysats de protéines cationiques, des polyalkylèneimines, , des polymères contenant des motifs vinylpyridine ou vinylpyridinium, des condensats de polyamines et d'épichlorhydrine, des polyuréylènes quaternaires et les dérivés de la chitine;

15. Composition selon l'une quelconque des revendications précédentes, **caractérisée par le fait que** ledit polymère cationique est choisi parmi les cyclopolymères cationiques, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, et le pyrrolidone-carboxylate de chitosane et leurs mélanges.

16. Composition selon l'une quelconque des revendications 12 à 15, **caractérisée en ce que** le ou les polymères cationiques représentent de 0,001 % à 20 % en poids, de préférence de 0,01 % à 10% en poids par rapport au poids total de la composition finale.

17. Composition selon l'une quelconque des revendications précédentes, **caractérisée en ce que** la composition comprend en outre une silicone, préférence une silicone aminée.

18. Composition selon la revendication précédente, **caractérisée en ce que** la silicone est choisie parmi les amodiméthicones et les triméthylsilylamodiméthicones, les silicones à groupements ammonium quaternaire.

19. Composition selon l'une quelconque des revendications 17 et 18, **caractérisée en ce que** la silicone est présente dans la composition à une teneur allant de 0,01 à 6%, de préférence de 0,1 à 3%, et encore de préférence de 0,4 à 2% en poids du poids total de la composition.

20. Composition selon l'une quelconque des revendications précédentes, **caractérisée en ce que** le milieu cosmétiquement acceptable est constitué par de l'eau ou par un ou plusieurs solvants cosmétiquement acceptables ou par des mélanges eau-solvant(s).

21. Composition selon la revendication précédente, **caractérisée en ce que** le solvant cosmétiquement acceptable est choisi parmi l'éthanol et l'isopropanol.

22. Composition selon l'une quelconque des revendications précédentes, **caractérisée en ce qu'**elle contient en outre au moins un ou plusieurs additifs choisis parmi les parfums, les filtres UV, les tensioactifs non ioniques, anioniques, amphotères ou zwitterioniques, les conservateurs, les protéines, les vitamines, les provitamines, les polymères non ioniques, anioniques, amphotères ou zwitterioniques, les huiles minérales, végétales ou synthétiques, les agents antipelliculaires, les agents anti-chute, les colorants, les pigments, les réducteurs, les silicones, les cires végétales, les céramides, les polyols autres que ceux de l'invention.

23. Utilisation d'une composition selon l'une quelconque des revendications 1 à 22 pour le traitement cosmétique des cheveux et du cuir chevelu.

24. Procédé de traitement cosmétique des cheveux, **caractérisé en ce qu'**on applique sur les cheveux une composition cosmétique selon l'une des revendications 1 à 22.

25. Procédé selon la revendication 24, **caractérisé en ce que** l'application s'effectue après un shampooing.

## Claims

1. Cosmetic composition, notably for the hair, **characterized in that** it comprises, in a cosmetically acceptable medium, at least one cationic surfactant selected from the quaternary ammonium salts, at least one fatty alcohol, optionally oxyethylenated or glycerolated, at least one non-siliconized cationic polymer and at least one diol having 6 carbon atoms, the total concentration of fatty alcohol(s) being greater than or equal to 4 wt.% relative to the total weight of the composition, the ratio of the concentrations by weight of cationic surfactant to diol being greater than or equal to 1.

2. Composition according to Claim 1, **characterized in that** the quaternary ammonium salts are selected from:
- those having the following general formula (V): 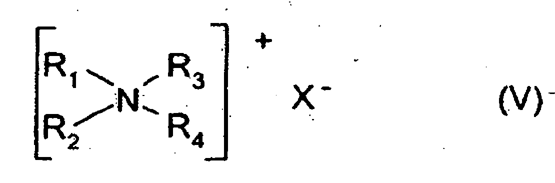
in which the symbols R₁ to R₄, which can be identical or different, represent a linear or branched aliphatic radical having from 1 to 30 carbon atoms, or an aromatic radical such as aryl or alkaryl; X⁻ is an anion selected from the group comprising the halides, phosphates, acetates, lactates, alkyl(C₂-C₆)sulphates, alkyl or alkaryl sulphonates;
- the quaternary ammonium salts of imidazoline of the following formula (VI): 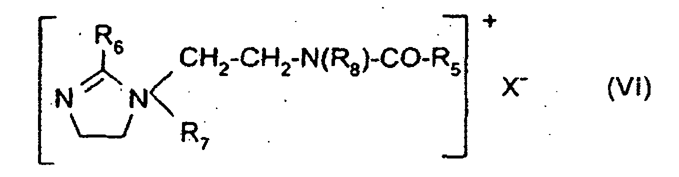
in which R₅ represents an alkenyl or alkyl radical with from 8 to 30 carbon atoms, R₆ represents a hydrogen atom, a C₁-C₄ alkyl radical or an alkenyl or alkyl radical having from 8 to 30 carbon atoms, R₇ represents a C₁-C₄ alkyl radical, R₈ represents a hydrogen atom, a C₁-C₄ alkyl radical, X is an anion selected from the group comprising the halides, phosphates, acetates, lactates, alkyl sulphates, alkyl or alkaryl sulphonates,
- the quaternary diammonium salts of formula (VII): 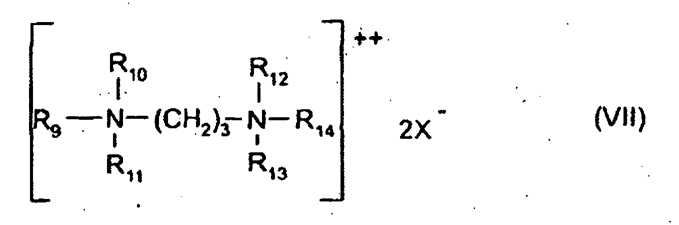
in which R₉ denotes an aliphatic radical having from 16 to 30 carbon atoms, R₁₀, R₁₁, R₁₂, R₁₃ and R₁₄, which may be identical or different, are selected from hydrogen or an alkyl radical having from 1 to 4 carbon atoms, and X⁻ is an anion selected from the group comprising the halides, acetates, phosphates, nitrates, ethyl sulphates and methyl sulphates;
- the quaternary ammonium salts containing at least one ester function, of the following formula (VIII): 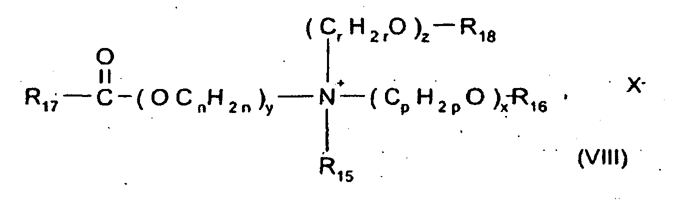
in which:
R₁₅ is selected from the C₁-C₆ alkyl radicals and the C₁-C₆ hydroxyalkyl or dihydroxyalkyl radicals;
R₁₆ is selected from:
- the radical 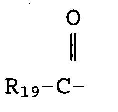
- the linear or branched, saturated or unsaturated C₁-C₂₂ hydrocarbon radicals R₂₀,
- the hydrogen atom,
R₁₇ is selected from:
- the radical 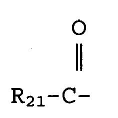
- the linear or branched, saturated or unsaturated C₁-C₆ hydrocarbon radicals R₂₂,
- the hydrogen atom,
R₁₇, R₁₉ and R₂₁, which may be identical or different, are selected from the linear or branched, saturated or unsaturated C₇-C₂₁ hydrocarbon radicals;
r, n and p, which may be identical or different, are integers from 2 to 6;
y is an integer from 1 to 10;
x and z, which may be identical or different, are integers from 0 to 10;
X⁻ is a simple or complex, organic or inorganic anion;
provided that the sum x + y + z has a value from 1 to 15, and that when x has the value 0, R₁₆ denotes R₂₀, and that when z has the value 0, R₁₈ denotes R₂₂.

3. Composition according to Claim 2, **characterized in that** the compound of formula (VIII) is selected from the salts of diacyloxyethyldimethylammonium, of diacyloxyethyl-hydroxyethylmethylammonium, of monoacyloxyethyl-dihydroxyethylmethylammonium, of triacyloxyethyl-methylammonium, of monoacyloxyethyl-hydroxyethyl-dimethylammonium and mixtures thereof.

4. Composition according to Claim 2, **characterized in that** the surfactant of formula (V) is selected from the salts of dipalmitoyl ethylhydroxyethyl-methylammonium, the salts of behenyltrimethylammonium, of distearyl-dimethylammonium, of cetyl trimethylammonium, of benzyldimethylstearylammonium, the salts of palmitylamidopropyltrimethylammonium, the salts of stearamido-propyl dimethyl(myristylacetate)-ammonium.

5. Composition according to Claim 1, **characterized in that** the cationic surfactant is selected from behenyl trimethylammonium chloride, cetyl trimethylammonium chloride, quaternium-83, behenylamidopropyl-2,3-dihydroxypropyl dimethyl ammonium chloride and palmitylamidopropyltrimethylammonium chloride.

6. Composition according to any one of the preceding claims, **characterized in that** the cationic surfactant is present in the composition at a content ranging from 0.01 to 10 wit.%, preferably from 0.1 to 5 wt.%, and more preferably from 0.2 to 4 wt.% of the total weight of the composition.

7. Composition according to any one of the preceding claims, **characterized in that** the fatty alcohol is linear or branched, saturated or unsaturated.

8. Composition according to Claim 7, **characterized in that** the fatty alcohol has the structure R-OH, in which R denotes a saturated or unsaturated, linear or branched radical, having from 8 to 40 carbon atoms and preferably from 8 to 30.

9. Composition according to Claim 8, **characterized in that** the fatty alcohol is selected from cetyl alcohol, stearyl alcohol, oleic alcohol and mixtures thereof.

10. Composition according to any one of the preceding claims, **characterized in that** the fatty alcohol is solid or pasty at room temperature.

11. Composition according to any one of the preceding claims, **characterized in that** the fatty alcohol is present in the composition at a content ranging from 4 to 15 wt.%, preferably from 4.5 to 10 wt.%, more preferably from 4.5 to 8 wt.% of the total weight of the composition.

12. Composition according to any one of the preceding claims, **characterized in that** the diol is selected from hexanediol-1,6, dipropylene glycol and mixtures thereof.

13. Composition according to any one of the preceding claims, **characterized in that** the diol is present in the composition at a content ranging from 0.1 to 10 wt.%, preferably from 0.25 to 5 wt.% and more preferably from 0.5 to 3 wt.% of the total weight of the composition.

14. Composition according to any one of Claims 1 to 13, **characterized in that** the cationic polymer is selected from:
(1) the homopolymers or copolymers derived from acrylic or methacrylic esters or amides and having at least one of the units in the following formulae: ' 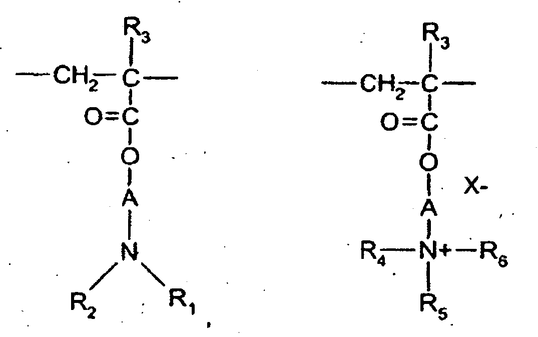 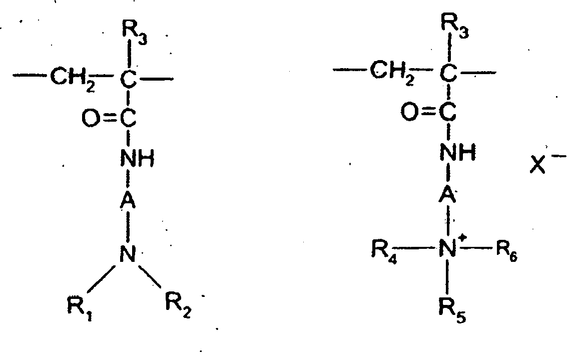 in which:
R₃, which may be identical or different, denote a hydrogen atom or a CH₃ radical;
A, which may be identical or different, represent a linear or branched alkyl group with 1 to 6 carbon atoms, preferably 2 or 3 carbon atoms or a hydroxyalkyl group with 1 to 4 carbon atoms;
R₄, R₅, R₆, which may be identical or different, represent an alkyl group having from 1 to 18 carbon atoms or a benzyl radical and preferably an alkyl group having from 1 to 6 carbon atoms;
R₁ and R₂, which may be identical or different, represent hydrogen or an alkyl group having from 1 to 6 carbon atoms and preferably methyl or ethyl;
X denotes an anion derived from an organic or mineral acid such as a methosulphate anion or a halide such as chloride or bromide.
(2) the cationic polysaccharides
(3) the polymers constituted of piperazinyl units and divalent linear or branched alkylene or hydroxyalkylene radicals, optionally interrupted by oxygen, sulphur, or nitrogen atoms or by aromatic or heterocyclic rings, as well as the products of oxidation and/or of quaternization of these polymers
(4) the water-soluble polyaminoamides prepared in particular by polycondensation of an acidic compound with a polyamine
(5) the derivatives of polyaminoamides resulting from the condensation of polyalkylene polyamines with polycarboxylic acids followed by alkylation by bifunctional agents
(6) the polymers obtained by reaction of a polyalkylene polyamine having two primary amine groups and at least one secondary amine group with a dicarboxylic acid
(7) the alkyl diallyl amine or dialkyl diallyl ammonium cyclopolymers
(8) the quaternary diammonium polymers containing repeating units corresponding to the formula: 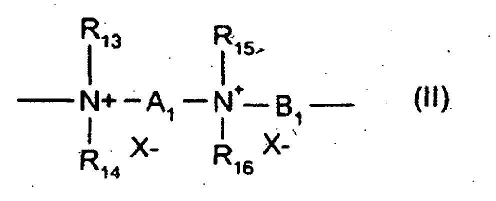 and in said formula (II):
R₁₃, R₁₄, R₁₅ and R₁₆, which may be identical or different, represent aliphatic, alicyclic, or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second heteroatom other than nitrogen or alternatively R₁₃, R₁₄, R₁₅ and R₁₆ represent a linear or branched C₁-C₆ alkyl radical substituted with a nitrile, ester, acyl, amide or -CO-O-R₁₇-D or CO-NH-R₁₇-D group, where R₁₇ is an alkylene and D is a quaternary ammonium group;
A₁ and B₁ represent polymethylene groups containing from 2 to 20 carbon atoms which can be linear or branched, saturated or unsaturated, and can contain, attached to or intercalated in the main chain, one or more aromatic rings, or one or more oxygen or sulphur atoms or sulphoxide, sulphone, disulphide, amino, alkylamino, hydroxyl, quaternary ammonium, ureido, amide or ester groups, and
X⁻ denotes an anion derived from an organic or mineral acid;
A₁, R₁₃ and R₁₅ can form, with the two nitrogen atoms to which they are attached, a piperazine ring; moreover, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
- [CH₂-CH(CH₃)-O]_{y}-CH₂-CH(CH₃)-
where x and y denote an integer from 1 to 4, representing a uniquely defined degree of polymerization or any number from 1 to 4 representing an average degree of polymerization;
b) a bis-secondary diamine residue such as a derivative of piperazine;
c) a bis-primary diamine residue of formula: -NH-Y-NH-, where Y denotes a linear or branched hydrocarbon radical, or alternatively the divalent radical
-CH₂-CH₂-S-S-CH₂-CH₂- ;
d) a ureylene group of formula: -NH-CO-NH-;
X⁻ is an anion
(9) the quaternary polyammonium polymers comprising units of formula (III): 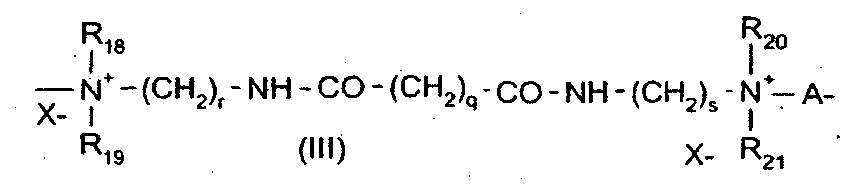 in which:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, provided that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X⁻ denotes an anion such as a halide,
A denotes a radical of a dihalide or preferably represents -CH₂-CH₂-O-CH₂-CH₂-,
(10) the quaternary polymers of vinyl pyrrolidone and of vinyl imidazole,
(11) the crosslinked polymers of methacryloyloxyalkyl (C₁-C₄) trialkyl (C₁-C₄) ammonium salts,
(12) cationic proteins or hydrolysates of cationic proteins, polyalkylene-imines, polymers containing vinyl pyridine or vinyl pyridinium units, condensates of polyamines and of epichlorohydrin, quaternary polyureylenes and derivatives of chitin;

15. Composition according to any one of the preceding claims, **characterized in that** said cationic polymer is selected from the cationic cyclopolymers, the quaternary polymers of vinyl pyrrolidone and of vinyl imidazole, the homopolymers or crosslinked copolymers of methacryloyloxyalkyl(C₁-C₄) trialkyl(C₁-C₄)ammonium salts, and the pyrrolidone-carboxylate of chitosan and mixtures thereof.

16. Composition according to any one of Claims 12 to 15, **characterized in that** the cationic polymer or polymers represent from 0.001 to 20 wt.%, preferably from 0.01 to 10 wt.% relative to the total weight of the final composition.

17. Composition according to any one of the preceding claims, **characterized in that** the composition additionally comprises a silicone, preferably an aminated silicone.

18. Composition according to the preceding claim, **characterized in that** the silicone is selected from the amodimethicones and the trimethylsilylamodimethicones, the silicones with quaternary ammonium groups.

19. Composition according to either of Claims 17 and 18, **characterized in that** the silicone is present in the composition at a content ranging from 0.01 to 6 wt.%, preferably from 0.1 to 3 wt.%, and more preferably from 0.4 to 2 wt.% of the total weight of the composition.

20. Composition according to any one of the preceding claims, **characterized in that** the cosmetically acceptable medium is constituted of water or of one or more cosmetically acceptable solvents or of water-solvent(s) mixtures.

21. Composition according to the preceding claim, **characterized in that** the cosmetically acceptable solvent is selected from ethanol and isopropanol.

22. Composition according to any one of the preceding claims, **characterized in that** it additionally contains at least one or more additives selected from perfumes, UV filters, nonionic, anionic, amphoteric or zwitterionic surfactants, preservatives, proteins, vitamins, provitamins, nonionic, anionic, amphoteric or zwitterionic polymers, mineral, vegetable or synthetic oils, anti-dandruff agents, agents against hair loss, dyes, pigments, reducing agents, silicones, vegetable waxes, ceramides, polyols other than those of the invention.

23. Use of a composition according to any one of Claims 1 to 22 for the cosmetic treatment of the hair and of the scalp.

24. Method of cosmetic treatment of the hair, **characterized in that** a cosmetic composition according to one of Claims 1 to 22 is applied on the hair.

25. Method according to Claim 24, **characterized in that** application is carried out after shampooing.

## Patentansprüche

1. Kosmetische Zusammensetzung, insbesondere für die Haare, **dadurch gekennzeichnet, dass** sie in einem kosmetisch akzeptablen Medium enthält:
mindestens ein kationisches Tensid, das unter quaternären Ammoniumsalzen ausgewählt ist, mindestens einen Fettalkohol, der gegebenenfalls oxyethyleniert oder glyceryliert ist, mindestens ein kationisches, nicht siliconhaltiges Polymer und mindestens ein Diol mit 6 Kohlenstoffatomen,
wobei die Gesamtkonzentration an Fettalkohol(en) größer als oder gleich 4 Gew.-% ist, bezogen auf das Gesamtgewicht der Zusammensetzung, und das gewichtsbezogene Konzentrationsverhältnis von kationischem Tensid zu Diol größer als oder gleich 1 ist.

2. Zusammensetzung nach Anspruch 1, **dadurch gekennzeichnet, dass** die quaternären Ammoniumsalze ausgewählt sind unter:
- quaternären Ammoniumsalzen der nachstehenden allgemeinen Formel (V): 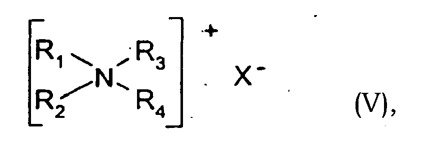 worin bedeuten:
R₁ bis R₄, die gleich oder verschieden sein können, eine geradkettige oder verzweigte aliphatische Gruppe mit 1 bis 30
Kohlenstoffatomen oder eine aromatische Gruppe wie Aryl oder Alkylaryl und
X⁻ ein Anion, das ausgewählt ist aus der Gruppe der Halogenide, Phosphate, Acetate, Lactate, C₂-C₆-Alkylsulfate, Alkylsulfonate oder Alkylarylsulfonate;
- quaternären Ammoniumsalzen von Imidazolinen der nachstehenden Formel (VI) 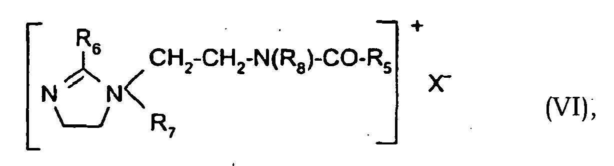 worin bedeuten:
R₅ eine Alkenylgruppe oder Alkylgruppe mit 8 bis 30 Kohlenstoffatomen,
R₆ ein Wasserstoffatom, eine C₁-C₄-Alkylgruppe oder eine Alkenylgruppe oder Alkylgruppe mit 8 bis 30 Kohlenstoffatomen,
R₇ eine C₁-C₄-Alkylgruppe,
R₈ ein Wasserstoffatom oder eine C₁-C₄-Alkylgruppe und
X⁻ ein Anion, das ausgewählt ist aus der Gruppe der Halogenide, Phosphate, Acetate, Lactate, Alkylsulfate, Alkylsulfonate oder Alkylarylsulfonate;
- quaternären Diammoniumsalzen der Formel (VII) 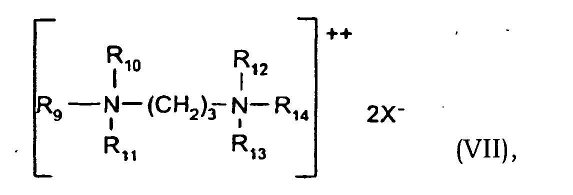 worin bedeuten:
R₉ eine aliphatische Gruppe mit 16 bis 30 Kohlenstoffatomen, R₁₀, R₁₁, R₁₂, R₁₃ und R₁₄, die gleich oder verschieden sind, Wasserstoff oder eine Alkylgruppe mit 1 bis 4 Kohlenstoffatomen,
und
X⁻ ein Anion, das ausgewählt ist aus der Gruppe der Halogenide, Acetate, Phosphate, Nitrate, Ethylsulfate und Methylsulfate;
- quaternären Ammoniumsalzen, die mindestens eine Esterfunktion enthalten, der nachstehenden Formel (VIII) 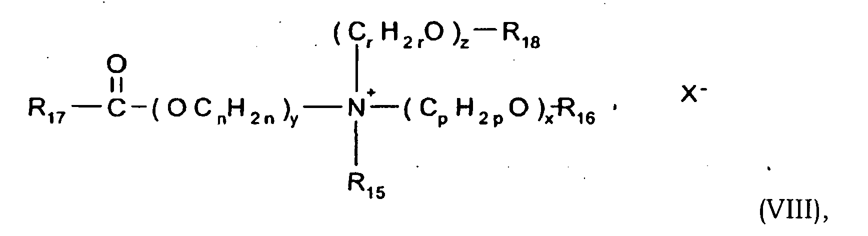
worin bedeuten:
R₁₅ eine Gruppe, die unter C₁-C₆-Alkylgruppen und C₁-C₆-Hydroxyalkylgruppen oder C₁-C₆-Dihydroxyalkylgruppen ausgewählt ist,
R₁₆ einen Substituenten, der ausgewählt ist unter:
- der Gruppe 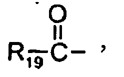
- geradkettigen oder verzweigten, gesättigten oder ungesättigten C₁-C₂₂-Kohlenwasserstoffgruppen R₂₀,
- einem Wasserstoffatom,
R₁₇ einen Substituenten, der ausgewählt ist unter:
- der Gruppe 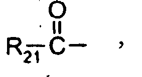
- den geradkettigen oder verzweigten, gesättigten oder ungesättigten C₁-C₆-Kohlenwasserstoffgruppen R₂₂,
- einem Wasserstoffatom,
R₁₇, R₁₉ und R₂₁, die gleich oder verschieden sind, eine
Gruppe, die ausgewählt ist unter geradkettigen oder verzweigten, gesättigten oder ungesättigten C₇-C₂₁-Kohlenwasserstoffgruppen,
r, n und p, die gleich oder verschieden sind, ganze Zahlen von 2 bis 6;
y eine ganze Zahl von 1 bis 10,
x und z, die gleich oder verschieden sind, ganze Zahlen von 0 bis 10
und
X⁻ ein einfaches oder komplexes organisches oder anorganisches Anion,
mit der Maßgabe, dass die Summe x + y + z im Bereich von 1 bis 15 liegt, R₁₆ dann, wenn x gleich 0 ist, R₂₀ bedeutet und R₁₈ dann, wenn z gleich 0 ist, R₂₂ bedeutet.

3. Zusammensetzung nach Anspruch 2, **dadurch gekennzeichnet, dass** die Verbindung der Formel (VIII) ausgewählt ist unter den Diacyloxyethyldimethylammoniumsalzen, den Diacyloxyethylhydroxyethylmethylammoniumsalzen, den Monoacyloxyethyldihydroxyethylmethylammoniumsalzen, den Triacyloxyethylmethylammoniumsalzen und den Monoacyloxyethylhydroxyethyldimethylammoniumsalzen sowie den Gemischen dieser Verbindungen.

4. Zusammensetzung nach Anspruch 2, **dadurch gekennzeichnet, dass** das Tensid der Formel (V) ausgewählt ist unter den Dipalmitoylethylhydroxyethylmethylammoniumsalzen, den Behenyltrimethylammoniumsalzen, den Distearyldimethylammoniumsalzen, den Cetyltrimethylammoniumsalzen, den Benzyldimethylstearylammoniumsalzen, den Palmitylamidopropyltrimethylammoniumsalzen und den Stearamidopropyldimethyl(myristylacetat)-ammoniumsalzen.

5. Zusammensetzung nach Anspruch 1, **dadurch gekennzeichnet, dass** das kationische Tensid ausgewählt ist unter Behenyltrimethylammoniumchlorid, Cetyltrimethylammoniumchlorid, Quaternium-83, Behenylamidopropyl-2,3-dihydroxypropyldimethylammoniumchlorid und Palmitylamidopropyltrimethylammoniumchlorid.

6. Zusammensetzung nach einem der vorhergehenden Ansprüche, **dadurch gekennzeichnet, dass** das kationische Tensid in der Zusammensetzung in einem Mengenanteil von 0,01 bis 10 Gew.-%, bevorzugt in einem Mengenanteil von 0,1 bis 5 Gew.-% und noch bevorzugter in einem Mengenanteil von 0,2 bis 4 Gew.-% vorliegt, bezogen auf das Gesamtgewicht der Zusammensetzung.

7. Zusammensetzung nach einem der vorhergehenden Ansprüche, **dadurch gekennzeichnet, dass** der Fettalkohol geradkettig oder verzweigt, gesättigt oder ungesättigt ist.

8. Zusammensetzung nach Anspruch 7, **dadurch gekennzeichnet, dass** der Fettalkohol die Formel R-OH aufweist, in der R eine gesättigte oder ungesättigte, geradkettige oder verzweigte Gruppe mit 8 bis 40 Kohlenstoffatomen und bevorzugt mit 8 bis 30 Kohlenstoffatomen bedeutet.

9. Zusammensetzung nach Anspruch 8, **dadurch gekennzeichnet, dass** der Fettalkohol unter Cetylalkohol, Stearylalkohol und Oleylalkohol sowie Gemischen dieser Verbindungen ausgewählt ist.

10. Zusammensetzung nach einem der vorhergehenden Ansprüche, **dadurch gekennzeichnet, dass** der Fettalkohol bei Umgebungstemperatur fest oder pastos ist.

11. Zusammensetzung nach einem der vorhergehenden Ansprüche, **dadurch gekennzeichnet, dass** der Fettalkohol in der Zusammensetzung in einem Mengenanteil von 4 bis 15 Gew.-%, bevorzugt in einem Mengenanteil von 4,5 bis 10 Gew.-% und noch bevorzugter in einem Mengenanteil von 4,5 bis 8 Gew.-% vorliegt, bezogen auf das Gesamtgewicht der Zusammensetzung.

12. Zusammensetzung nach einem der vorhergehenden Ansprüche, **dadurch gekennzeichnet, dass** das Diol unter 1,6-Hexandiol und Dipropylenglycol sowie Gemischen dieser Verbindungen ausgewählt ist.

13. Zusammensetzung nach einem der vorhergehenden Ansprüche, **dadurch gekennzeichnet, dass** das Diol in der Zusammensetzung in einem Mengenanteil von 0,1 bis 10 Gew.-%, bevorzugt in einem Mengenanteil von 0,25 bis 5 Gew.-% und noch bevorzugter in einem Mengenanteil von 0,5 bis 3 Gew.-% vorliegt, bezogen auf das Gesamtgewicht der Zusammensetzung.

14. Zusammensetzung nach einem der Ansprüche 1 bis 13, **dadurch gekennzeichnet, dass** das kationische Polymer ausgewählt ist unter:
(1) Homopolymeren oder Copolymeren, die von Estern oder Amiden von Acrylsäuren oder Methacrylsäuren abgeleitet sind und mindestens eine der Einheiten der nachstehenden Formeln enthalten: 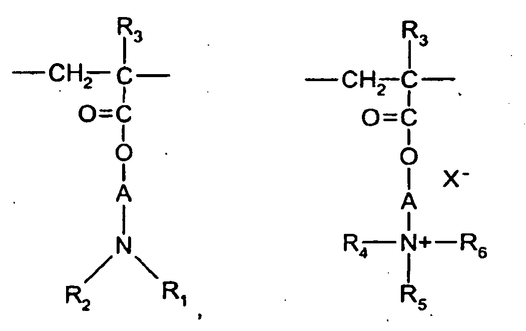 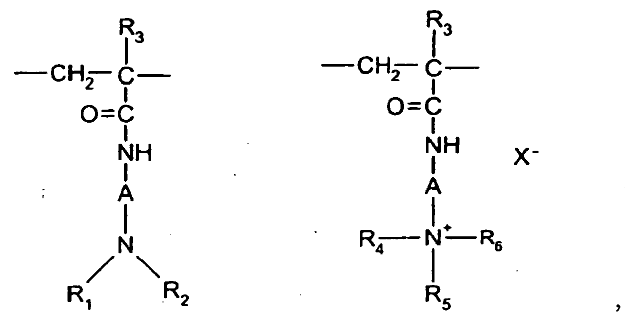 worin bedeuten:
R₃, die gleich oder verschieden sind, ein Wasserstoffatom oder eine Gruppe CH₃,
A, die gleich oder verschieden sind, eine geradkettige oder verzweigte Alkylgruppe mit 1 bis 6 Kohlenstoffatomen und bevorzugt 2 oder 3 Kohlenstoffatomen oder eine Hydroxyalkylgruppe mit 1 bis 4 Kohlenstoffatomen;
R₄, R₅ und R₆, die gleich oder verschieden sind, eine Alkylgruppe mit 1 bis 18 Kohlenstoffatomen oder eine Benzylgruppe und bevorzugt eine Alkylgruppe mit 1 bis 6 Kohlenstoffatomen;
R₁ und R₂, die gleich oder verschieden sind, Wasserstoff oder eine Alkylgruppe mit 1 bis 6 Kohlenstoffatomen und bevorzugt Methyl oder Ethyl;
X ein von einer anorganischen Säure oder einer organischen Säure abgeleitetes Anion, wie etwa ein Methosulfat-Anion oder ein Halogenid, wie das Chlorid oder Bromid;
(2) kationischen Polysacchariden;
(3) Polymeren, die aus Piperazinyl-Einheiten und zweiwertigen Alkylen- oder Hydroxyalkylen-Einheiten mit geraden oder verzweigten Ketten bestehen, die gegebenenfalls durch Sauerstoffatome, Schwefelatome, Stickstoffatome oder durch aromatische oder heterocyclische Ringe unterbrochen sind, sowie die Oxidationsprodukte und/oder Quaternisierungsprodukte dieser Polymeren;
(4) wasserlöslichen Polyaminoamiden, die insbesondere durch Polykondensation einer sauren Verbindung mit einem Polyamin hergestellt sind;
(5) Derivaten von Polyaminoamiden, die aus der Kondensation von Polyalkylenpolyaminen mit Polycarbonsäuren und anschließende Alkylierung mit bifunktionellen Agentien resultieren;
(6) Polymeren, die durch Umsetzung eines Polyalkylenpolyamins mit zwei primären Aminogruppen und mindestens einer sekundären Aminogruppe mit einer Dicarbonsäure erhalten sind;
(7) Cyclopolymeren von Alkyldiallylamin oder Dialkyldiallylammonium-Verbindungen;
(8) Polymeren von quaternären Diammoniumverbindungen mit wiederkehrenden Einheiten der Formel 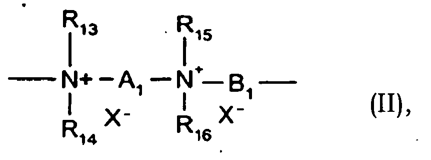 worin bedeuten:
R₁₃, R₁₄, R₁₅ und R₁₆, die gleich oder verschieden sind, aliphatische, alicyclische oder arylaliphatische Gruppen mit 1 bis 20 Kohlenstoffatomen oder niedere hydroxyalkylaliphatische Gruppen, oder R₁₃, R₁₄, R₁₅ und R₁₆ bilden zusammen oder getrennt mit den Stickstoffatomen, an denen sie gebunden sind, Heterocyclen, die gegebenenfalls ein zweites Heteroatom enthalten, das von Stickstoff verschieden ist,
oder R₁₃, R₁₄, R₁₅ und R₁₆ stellen eine geradkettige oder verzweigte C₁-C₆-Alkylgruppe dar, die mit einer Nitrilgruppe, einer Estergruppe, einer Acylgruppe, einer Amidgruppe oder einer Gruppe -CO-O-R₁₇-D oder CO-NH-R₁₇-D substituiert ist, worin R₁₇ eine Alkylengruppe und D eine quaternäre Ammoniumgruppe bedeuten,
A₁ und B₁ Polymethylengruppen mit 2 bis 20 Kohlenstoffatomen, die geradkettig oder verzweigt, gesättigt oder ungesättigt sein können und daran gebunden oder in die Hauptkette eingeschaltet einen oder mehrere aromatische Ringe oder ein oder mehrere Sauerstoffatome, Schwefelatome oder Sulfoxidgruppen, Sulfongruppen, Disulfidgruppen, Aminogruppen, Alkylaminogruppen, Hydroxylgruppen, quaternäre Ammoniumgruppen, Ureidogruppen, Amidgruppen oder Estergruppen enthalten können, und
X⁻ ein von einer anorganischen oder organischen Säure abgeleitetes Anion,
wobei A₁, R₁₃ und R₁₅ mit den beiden Stickstoffatomen, an denen sie gebunden sind, einen Piperazinring bilden können,
wobei ferner dann, wenn A₁ eine geradkettige oder verzweigte, gesättigte oder ungesättigte Alkylengruppe oder Hydroxyalkylengruppe bedeutet, B₁ auch eine Gruppe (CH₂)ₙ-CO-D-OC-(CH₂)ₙ- bedeuten kann,
worin D bedeutet:
a) eine Glycolgruppe der Formel -O-Z-O-, worin Z eine geradkettige oder verzweigte Kohlenwasserstoffgruppe oder eine Gruppe einer der nachstehenden Formeln bedeutet:
- (CH₂-CH₂-O)x-CH₂-CH₂-
- [CH₂-CH(CH₃)-O]y-CH₂-CH(CH₃)-,
worin x und y eine ganze Zahl von 1 bis 4, die einen definierten und einzigen Polymerisationsgrad darstellt, oder eine beliebige Zahl von 1 bis 4 darstellen, die einem mittleren Polymerisationsgrad entspricht;
b) einen bis-sekundären Diaminrest wie etwa ein Piperazinderivat;
c) einen bis-primären Diaminrest der Formel -NH-Y-NH-, worin Y eine geradkettige oder verzweigte Kohlenwasserstoffgruppe oder die zweiwertige Gruppe -CH₂-CH₂-S-S-CH₂-CH₂- bedeutet;
d) eine Ureylengruppe der Formel -NH-CO--NH- , und
X⁻ ein Anion;
(9) quaternären Polyammonium-Polymeren mit Einheiten der Formel 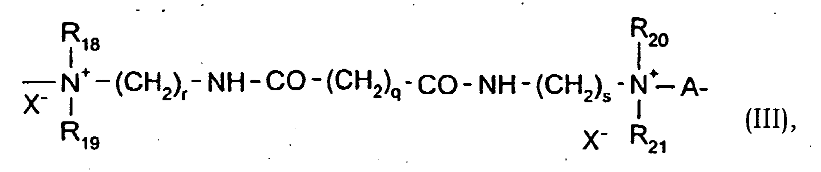 worin bedeuten '
R₁₈, R₁₉, R₂₀ und R₂₁, die gleich oder verschieden sind, ein Wasserstoffatom oder Methyl, Ethyl, Propyl, β-Hydroxyethyl, β-Hydroxypropyl oder -CH₂CH₂(OCH₂CH₂)p-OH, worin p gleich 0 oder eine ganze Zahl von 1 bis 6 bedeutet, mit der Maßgabe, dass R₁₈, R₁₉, R₂₀ und R₂₁ nicht gleichzeitig ein Wasserstoffatom bedeuten,
r und s, die gleich oder verschieden sind, ganze Zahlen von 1 bis 6,
q 0 oder eine ganze Zahl von 1 bis 34,
X⁻ ein Anion, wie etwa ein Halogenid,
und
A eine Dihalogenidgruppe oder bevorzugt -CH₂-CH₂-O-CH₂-CH₂-;
(10) quaternären Polymeren von Vinylpyrrolidon und Vinylimidazol;
(11) vernetzten Polymeren von Methacryloyloxy-(C₁-C₄)-alkyl-(C₁-C₄)-trialkyl-ammoniumsalzen;
(12) kationischen Proteinen oder kationischen Proteinhydrolysaten, Polyalkyleniminen, Polymeren, die Vinylpyridin- oder Vinylpyridinium-Einheiten enthalten, Kondensationsprodukten von Polyaminen mit Epichlorhydrin, quaternären Polyureylenen und Derivaten von Chitin.

15. Zusammensetzung nach einem der vorhergehenden Ansprüche, **dadurch gekennzeichnet, dass** das kationische Polymer ausgewählt ist unter kationischen Cyclopolymeren, quaternären Polymeren von Vinylpyrrolidon und Vinylimidazol, vernetzten Homopolymeren oder Copolymeren von Methacryloyloxy-(C₁-C₄)-alkyl-(C₁-C₄)-trialkyl-ammoniumsalzen und Chitosanpyrrolidoncarboxylat sowie Gemischen dieser Verbindungen.

16. Zusammensetzung nach einem der Ansprüche 12 bis 15, **dadurch gekennzeichnet, dass** das kationische Polymer oder die kationischen Polymeren in einem Mengenanteil von 0,001 bis 20 Gew.-%, bevorzugt in einem Mengenanteil von 0,01 bis 10 Gew.-% vorliegen, bezogen auf das Gesamtgewicht der fertigen Zusammensetzung.

17. Zusammensetzung nach einem der vorhergehenden Ansprüche, **dadurch gekennzeichnet, dass** die Zusammensetzung ferner ein Silicon enthält, bevorzugt ein Aminogruppen enthaltendes Silicon.

18. Zusammensetzung nach dem vorhergehenden Anspruch, **dadurch gekennzeichnet, dass** das Silicon unter Amodimethiconen und Trimethylsilylamodimethiconen und Siliconen mit quaternären Ammoniumgruppen ausgewählt ist.

19. Zusammensetzung nach einem der Ansprüche 17 und 18, **dadurch gekennzeichnet, dass** das Silicon in der Zusammensetzung in einem Mengenanteil von 0,01 bis 6 Gew.-%, bevorzugt in einem Mengenanteil von 0,1 bis 3 Gew.-% und noch bevorzugter in einem Mengenanteil von 0,4 bis 2 Gew.-% vorliegt, bezogen auf das Gesamtgewicht der Zusammensetzung.

20. Zusammensetzung nach einem der vorhergehenden Ansprüche, **dadurch gekennzeichnet, dass** das kosmetisch akzeptable Medium aus Wasser oder einem oder mehreren kosmetisch akzeptablen Lösungsmitteln oder Gemischen aus Wasser und einem oder mehreren Lösungsmitteln besteht.

21. Zusammensetzung nach dem vorhergehenden Anspruch, **dadurch gekennzeichnet, dass** das kosmetisch akzeptable Lösungsmittel unter Ethanol und Isopropanol ausgewählt ist.

22. Zusammensetzung nach einem der vorhergehenden Ansprüche, **dadurch gekennzeichnet, dass** sie ferner mindestens ein oder mehrere Additive enthält, die ausgewählt sind unter Duftstoffen, UV-Filtern, nichtionischen, anionischen, amphoteren oder zwitterionischen Tensiden, Konservierungsmitteln, Proteinen, Vitaminen, Provitaminen, nichtionischen, anionischen, amphoteren oder zwitterionischen Polymeren, Mineralölen, Pflanzenölen oder synthetischen Ölen, Mitteln gegen Schuppen, Mitteln gegen Haarausfall, Färbemitteln, Pigmenten, Reduktionsmitteln, Siliconen, pflanzlichen Wachsen, Ceramiden und Polyolen, die von denen der Erfindung verschieden sind.

23. Verwendung einer Zusammensetzung nach einem der Ansprüche 1 bis 22 zur kosmetischen Behandlung der Haare und der Kopfhaut.

24. Verfahren zur kosmetischen Behandlung der Haare, **dadurch gekennzeichnet, dass** eine kosmetische Zusammensetzung nach einem der Ansprüche 1 bis 22 auf die Haare aufgebracht wird.

25. Verfahren nach Anspruch 24, **dadurch gekennzeichnet, dass** das Aufbringen nach einem Shampoonieren vorgenommen wird.
